(19) Europäisches Patentamt
European Patent Office
Office européen des brevets

(11) **EP 3 622 944 A1**

(12) **EUROPEAN PATENT APPLICATION**
published in accordance with Art. 153(4) EPC

(43) Date of publication:
**18.03.2020 Bulletin 2020/12**

(51) Int Cl.:
**A61K 8/04** (2006.01)     **A61K 8/29** (2006.01)
**A61K 8/27** (2006.01)     **A61Q 17/04** (2006.01)

(21) Application number: **17909176.4**

(22) Date of filing: **11.05.2017**

(86) International application number:
**PCT/KR2017/004876**

(87) International publication number:
**WO 2018/207958 (15.11.2018 Gazette 2018/46)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(71) Applicant: **Cosmax, Inc.**
**Hwaseong-si, Gyeonggi-do 18622 (KR)**

(72) Inventors:
• **PARK, Sung Mi**
  **Hwaseong-si**
  **Gyeonggi-do 18475 (KR)**
• **PARK, Myeong Sam**
  **Seoul 04987 (KR)**
• **KIM, Hee Yong**
  **Anyang-si**
  **Gyeonggi-do 14125 (KR)**

(74) Representative: **Krauss, Jan**
**Boehmert & Boehmert**
**Anwaltspartnerschaft mbB**
**Pettenkoferstrasse 22**
**80336 München (DE)**

(54) **COSMETIC PRODUCT IMPREGNATED WITH COSMETIC COMPOSITION HAVING ULTRAVIOLET BLOCKING FUNCTION**

(57)     The present invention relates to a sunscreen cosmetic product including a foamed rubber sponge and a cosmetic composition impregnated in the foamed rubber sponge, wherein the cosmetic composition comprises only an inorganic ultraviolet blocking agent as an ultraviolet blocking agent. In the sunscreen cosmetic product including a foamed rubber sponge and a cosmetic composition impregnated in the foamed rubber sponge, when the cosmetic composition comprises only an inorganic ultraviolet blocking agent as an ultraviolet blocking agent, it is possible to realize the sunscreen effect of the content wholly even after impregnation, since the inorganic ultraviolet blocking agent is not adsorbed to the foamed rubber sponge but is dispensed with no loss caused by adsorption. In addition, it is possible to increase the usage efficiency by controlling the pore size of the foamed rubber sponge according to the present invention during foaming to improve the ratio of the amount that can be used by the consumers based on the amount of the content impregnated in the sponge.

**Description**

Technical Field]

The present invention relates to a cosmetic product impregnated with a cosmetic composition having an ultraviolet blocking function. More particularly, the present invention relates to a sunscreen cosmetic product including a foamed rubber sponge and a cosmetic composition impregnated in the foamed rubber sponge, wherein the cosmetic composition comprises only an inorganic ultraviolet blocking agent as an ultraviolet blocking agent.

Background Art]

Recently, as the amount of UV rays reaching the earth's surface has been increased due to ozone depletion caused by environmental pollution, many studies have been conducted about the effects of UV rays upon the human body. Most typically, many studies have been conducted about skin aging generated by continuous exposure to UV rays. As a primary method for delaying such a skin aging phenomenon, there is a method for reducing penetration of UV rays through the skin by using a sunscreen. In this context, more recently, many studies have been conducted about sunscreen cosmetic compositions for protecting the skin from UV rays (particularly, UV-A and UV-B). Sunscreens used currently in the field of cosmetics may be broadly classified into inorganic ultraviolet blocking agents and organic ultraviolet blocking agents. Inorganic ultraviolet blocking agents are not absorbed to the skin but merely function on the skin surface. Such inorganic ultraviolet blocking agents are materials having physical properties of reflecting and dispersing UV rays, and particular examples thereof include titanium dioxide, zinc oxide, or the like. Inorganic ultraviolet blocking agents are advantageous in that they have a high refractive index to provide an effect of scattering UV rays and cause low skin irritation, but are disadvantageous in that they cause a white cast phenomenon. Organic sunscreens are materials keeping solar light energy in their molecules and thus absorb UV rays and provide a sunscreen effect, and particular examples thereof include ethylhexylmethoxy cinnamate, octylmethoxy cinnamate, ethylhexyl salicylate, or the like. Unlike inorganic ultraviolet blocking agents, organic ultraviolet blocking agents cause no white case effect but cause skin irritation.

Formulations used for sunscreen cosmetic products include liquid, cream, sprays, stick formulations, or the like. However, liquid- and cream-type sunscreen cosmetic products show inconvenience of use, since they are taken by the hand and applied to the skin. Spray or stick formulations are limited in application, since it is difficult to apply such formulations to the face. To solve the above-mentioned problems, some formulations including porous sponge or porous foam impregnated with a cosmetic composition have been developed recently.

Korean Patent Publication No. 1542917 discloses a cosmetic product including sponge impregnated with a cosmetic composition, wherein the cosmetic composition includes an organic ultraviolet blocking agent only, or both an organic ultraviolet blocking agent and an inorganic ultraviolet blocking agent, as an ultraviolet blocking agent. However, in the case of an organic ultraviolet blocking agent, it is adsorbed to sponge by nature, and thus causes degradation of the sunscreen effect of the cosmetic composition dispensed from the sponge, as compared to the sunscreen effect of the cosmetic composition before impregnation, when the cosmetic composition impregnated in sponge is to be used by the consumers practically. In addition, in the case of conventional sponge made of rubber having a small pore size, there is a problem in that the usage efficiency of the cosmetic composition is low as compared to the amount of the cosmetic composition loaded in the sponge.

Under these circumstances, the present inventors have conducted intensive studies to overcome the above-mentioned problems according to the related art. As a result, we have found that when a sunscreen cosmetic product includes foamed rubber sponge having a controlled pore size and a cosmetic composition impregnated in the foamed rubber sponge, and the cosmetic composition comprises only an inorganic ultraviolet blocking agent as an ultraviolet blocking agent, it is possible to realize the sunscreen effect of the content wholly even after impregnation, since the inorganic ultraviolet blocking agent is not adsorbed to the foamed rubber sponge but is dispensed with no loss caused by adsorption. In addition, it is possible to increase the usage efficiency by controlling the pore size of the foamed rubber sponge according to the present invention during foaming to improve the ratio of the amount that can be used by the consumers based on the amount of the content impregnated in the sponge. The present invention is based on these findings.

**[Disclosure]**

[Technical Problem]

A technical problem to be solved by the present invention is to provide a cosmetic composition and cosmetic product which can realize the sunscreen effect of its content wholly even after the content is impregnated in sponge.

Another technical problem to be solved by the present invention is to increase the usage efficiency of the cosmetic composition based on the amount of the cosmetic composition loaded in the sponge by controlling the pore size of sponge made of rubber during foaming.

[Technical Solution]

In one general aspect, there is provided a sunscreen cosmetic product including a foamed rubber sponge and a cosmetic composition impregnated in the foamed rubber sponge, wherein the cosmetic composition comprises only an inorganic ultraviolet blocking agent as an ultraviolet blocking agent.

In general, a cosmetic product including sponge impregnated with a cosmetic composition having an ultraviolet blocking function according to the related art includes an organic ultraviolet blocking agent, or both an organic ultraviolet blocking agent and an inorganic ultraviolet blocking agent. In the case of an organic ultraviolet blocking agent, it is adsorbed to sponge by nature, and thus the amount of organic ultraviolet blocking agent dispensed from the sponge is smaller than the amount of organic ultraviolet blocking agent impregnated in the sponge, thereby providing low sun protection efficiency undesirably. To solve the above-mentioned problems, the present invention provides a sunscreen cosmetic product including a foamed rubber sponge impregnated with a cosmetic composition containing an inorganic ultraviolet blocking agent ingredient only, without any organic ultraviolet blocking agent.

According to Test Examples of the present invention, a sunscreen cosmetic product (Example 1) including foamed rubber sponge impregnated with a cosmetic composition containing an inorganic ultraviolet blocking agent only was compared with a cosmetic product (Comparative Example 1) including foamed rubber sponge impregnated with a cosmetic composition containing both an organic ultraviolet blocking agent and an inorganic ultraviolet blocking agent, in terms of the amount of cosmetic composition dispensed from the sponge based on the amount of cosmetic composition loaded in the sponge. Herein, the sponge used in Comparative Example 1 has the same pore size, density and hardness as the sponge used in Example 1. As a result, Example 1 showed a higher ratio of the amount dispensed from the sponge based on the amount loaded in the sponge, as compared to Comparative Example 1. Based on this, usage efficiency (dispensed amount/loaded amount (%)) was calculated. It can be seen that Example 1 shows higher usage efficiency. As can be seen from the above results, the sunscreen cosmetic product according to the present invention shows a higher sunscreen effect by virtue of such high dispensation of cosmetic composition.

In addition, according to Test Examples, a sunscreen cosmetic product (Example 1) including foamed rubber sponge impregnated with a cosmetic composition containing an inorganic ultraviolet blocking agent only was compared with a cosmetic product (Comparative Example 1-3) including foamed rubber sponge impregnated with a cosmetic composition containing both an organic ultraviolet blocking agent and an inorganic ultraviolet blocking agent, in terms of formulation stability. As a result, it can be seen that when using foamed rubber sponge having the same (Comparative Example 1) or a smaller (Comparative Example 2) pore size and density, the cosmetic product shows high formulation stability with no separation of content, like the cosmetic product according to the present invention. However, Comparative Example 3 using foamed rubber sponge having a larger pore size as compared to the present invention causes separation of the content. As can be seen from the above results, the foamed rubber sponge according to the present invention allows stable impregnation of the content to prevent separation of the content, and thus can retain formulation stability.

According to the present invention, the sunscreen cosmetic product is characterized in that it includes no organic ultraviolet blocking agent.

Since an organic ultraviolet blocking agent is adsorbed to sponge by nature, it has a limitation in efficient protection of UV rays. Thus, the cosmetic composition according to the present invention includes no organic ultraviolet blocking agent.

According to Examples, in the case of Comparative Examples (1-3) containing an organic ultraviolet blocking agent, it can be seen that the ratio of the amount of organic ultraviolet blocking agent dispensed from sponge based on the amount of organic ultraviolet blocking agent impregnated in sponge is low. On the contrary, in the case of an inorganic ultraviolet blocking agent, the ratio of the amount of inorganic ultraviolet blocking agent dispensed from sponge based on the amount of inorganic ultraviolet blocking agent impregnated in sponge is 100%. As can be seen from the results, the sunscreen cosmetic product including an inorganic ultraviolet blocking agent only according to the present invention allows whole dispensation of the inorganic ultraviolet blocking agent when the cosmetic product is used, wherein the inorganic ultraviolet blocking agent is not adsorbed to the sponge. Therefore, the sunscreen cosmetic product according to the present invention shows a higher sunscreen effect.

According to the present invention, the foamed rubber sponge has a pore size of 0.1-1.0 mm, density of 150-250 g/L and a hardness of 22 ± 5 based on Asker hardness F. When the pore size is smaller than the above-defined range, it is difficult to impregnate the sponge with the cosmetic composition and the usage efficiency based on the amount of cosmetic composition loaded in the sponge is low undesirably. When the pore size is larger than the above-defined range, the usage efficiency based on the amount of cosmetic composition loaded in the sponge is high, but such an excessively large pore size causes low content stability and results in a decrease in force by which the content is captured, so that the content may be dispensed even under low impact. When the density of foamed rubber sponge is less than the above-defined range, the cosmetic composition is taken in an excessively large amount to cause a decrease in usage efficiency. When the density is larger than the above-defined range, pores capable of supporting the content are insufficient so that the sponge may not be impregnated with the cosmetic composition effectively.

According to the present invention, the foamed rubber sponge is any foamed rubber sponge, as long as it can support the cosmetic composition including an inorganic ultraviolet blocking agent. Preferably, the foamed rubber sponge is styrene-butadiene rubber (SBR), butadiene rubber (BR), acrylonitrile rubber (NR) or acrylonitrile-butadiene rubber (NBR), and more preferably acrylonitrile-butadiene rubber (NBR).

According to the present invention, the cosmetic composition impregnated in the foamed rubber sponge has a viscosity of 4,000-15,000 cps. When the cosmetic composition has a viscosity less than 4,000 cps, the bulk-state content before impregnation is separated rapidly. Meanwhile, when the content has a viscosity higher than 15,000 cps, it is difficult to impregnate the sponge with the content, and the advantages of cushion, i.e. a thin and light feeling of use, may be degraded.

According to the present invention, the cosmetic composition is make-up base, blemish balm (BB) cream, foundation, primer or sunscreen.

The cosmetic composition according to the present invention may be prepared by the conventional methods known to those skilled in the art, considering the types and amounts of ingredients, formulations, or the like. In addition, impregnation of the foamed rubber sponge with the cosmetic composition may be carried out by the conventional methods known to those skilled in the art.

[Advantageous Effects]

As described hereinabove, the cosmetic product impregnated with a cosmetic composition having an ultraviolet blocking function according to the present invention comprises only an inorganic ultraviolet blocking agent as an ultraviolet blocking agent. Thus, when using the cosmetic product, the ratio of the amount of cosmetic composition dispensed from the foamed rubber sponge based on the amount of cosmetic composition impregnated in the foamed rubber sponge is high. In addition, since the inorganic ultraviolet blocking agent is not adsorbed to the foamed rubber sponge, it is possible to provide a significantly high sunscreen effect and high formulation stability.

[Best Mode]

Exemplary embodiments now will be described more fully hereinafter. The following exemplary embodiments are for illustrative purposes only and the scope of the present invention should not be construed as limited to the exemplary embodiments set forth therein.

The foundations of Example 1 and Comparative Examples 1-3 were prepared by using W/O (water-in-oil) emulsion type make-up cosmetic compositions according to the ingredients and amounts as shown in the following Table 1.

**Example 1: Preparation of Cosmetic Composition Including Only Inorganic Sunscreen**

Example 1 includes rubber sponge having a pore size of 0.1-1.0 mm and a density of 150-250 g/L and impregnated with a water-in-oil type cosmetic composition containing an inorganic ultraviolet blocking agent only. The water-in-oil type foundation was prepared as described hereinafter according to the composition and amounts as shown in Table 1.

The oil-phase ingredients and thickening agent were introduced to an oil-phase container and homogenized by heating to 70°C, and then the pigment was introduced thereto and dispersed therein. The aqueous phase ingredients were introduced to an aqueous phase container and dissolved completely by heating to 70°C under agitation. Then, the aqueous phase ingredients were introduced gradually to the oil-phase container in which the pigment was dispersed, and the resultant mixture was emulsified by using a homo-mixer to obtain a water-in-oil type low-viscosity sunscreen emulsion.

**Comparative Example 1: Preparation of Cosmetic Composition Including Inorganic Sunscreen and Organic Sunscreen**

Comparative Example 1 includes rubber sponge having a pore size of 0.1-1.0 mm and a density of 150-250 g/L and impregnated with a water-in-oil type cosmetic composition containing an inorganic ultraviolet blocking agent and an organic ultraviolet blocking agent. The water-in-oil type foundation was prepared in the same manner as Example 1 according to the composition and amounts as shown in Table 1.

**Comparative Example 2: Preparation of Cosmetic Composition Including Inorganic Sunscreen and Organic Sunscreen**

Comparative Example 2 includes rubber sponge having an average pore size of 0.15 mm and a density of 330 g/L and impregnated with a water-in-oil type cosmetic composition containing an inorganic ultraviolet blocking agent and an organic ultraviolet blocking agent. The water-in-oil type foundation was prepared in the same manner as Example 1 according to the composition and amounts as shown in Table 1.

**Comparative Example 3: Preparation of Cosmetic Composition Including Inorganic Sunscreen and Organic Sunscreen**

Comparative Example 3 includes rubber sponge having an average pore size of 1.2 mm and a density of 90 g/L and impregnated with a water-in-oil type cosmetic composition containing an inorganic ultraviolet blocking agent and an organic ultraviolet blocking agent. The water-in-oil type foundation was prepared in the same manner as Example 1 according to the composition and amounts as shown in Table 1.

[Table 1]

| | Name of ingredients | Content (weight %) | | | |
|---|---|---|---|---|---|
| | | Example1 | Comp.Ex.1 | Comp.Ex.2 | Comp.Ex.3 |
| Oil-phase ingredients | Cyclopentasiloxane | 25.0 | 14.5 | 14.5 | 14.5 |
| | Ethylhexylmethoxycinnamate | - | 7.5 | 7.5 | 7.5 |
| | Ethylhexylsalicylate | - | 4.0 | 4.0 | 4.0 |
| | Caprylic/Capric Triglycerides | 5.0 | 5.0 | 5.0 | 5.0 |
| | PEG-10 dimethicone | 3.0 | 3.0 | 3.0 | 3.0 |
| | Diphenylsiloxyphenyltrimethicone | 2.0 | 2.0 | 2.0 | 2.0 |
| | Dimethicone | 2.0 | 2.0 | 2.0 | 2.0 |
| | Sorbitan isostearate | 1.0 | 1.0 | 1.0 | 1.0 |
| Thickening agent | Disteadimonium hectorite | Sutable Amount | Sutable Amount | Sutable Amount | Sutable Amount |
| Pigment | Titanium dioxide | 4.0 | 4.0 | 4.0 | 4.0 |
| | Titanium dioxide Aluminum hydroxide Stearic acid | 9.0 | 9.0 | 9.0 | 9.0 |
| | Yellow iron oxide | 0.8 | 0.8 | 0.8 | 0.8 |
| | Red iron oxide | 0.2 | 0.2 | 0.2 | 0.2 |
| | Black iron oxide | 0.1 | 0.1 | 0.1 | 0.1 |
| | Mica | 4.0 | 4.0 | 4.0 | 4.0 |
| Aqueous phase ingredients | Purified water | to 100 | to 100 | to 100 | to 100 |
| | Glycerin | 5.0 | 5.0 | 5.0 | 5.0 |
| | Salt | 1.0 | 1.0 | 1.0 | 1.0 |
| | | | | | |
| Applied sponge | | NBR Pore size 0.5mm Density 200g/L | NBR Pore size 0.5mm Density 200g/L | NBR Pore size 0.15mm Density 330g/L | NBR Pore size 1.2mm Density 90g/L |

**Test Example 1: Determination of Dispensation/Impregnation of Cosmetic Composition**

In Test Example 1, each of the cosmetic products according to Example 1 and Comparative Examples 1-3 was impregnated in sponge in an amount of 14.5 g and stored in a constant-temperature container at 25°C for at least one day. The use of each cosmetic composition in each stage was measured as described hereinafter. The results are shown in the following Tables 2-4.

<Method for Measurement>

(Stage 1) The amount of cosmetic composition applied to white paper by using Ruby-Cell puff, until the white paper is little stained with the content.
(Stage 2) The amount of cosmetic composition applied to white paper by using Ruby-Cell puff after the sponge for impregnation is overturned, until the white paper is little stained with the content.
(Stage 3) The amount of cosmetic composition applied directly to white paper by using the sponge for impregnation removed from the product, until the white paper is little stained with the content.

[Table 2]

| Example1 | Applicator | Loaded amount (g) | Stage 1 dispensed amount (g) | Stage 2 dispensed amount (g) | Stage 3 dispensed amount (g) | Total dispense d amount (g) | Dispensed amount / Loaded amount (%) |
|---|---|---|---|---|---|---|---|
| Sample No.1 | Puff | 14.5 | 9.18 | 3.22 | 0.84 | 13.24 | 91.31 |
| Sample No.2 | Puff | 14.5 | 9.4 | 2.41 | 1.2 | 13.01 | 89.72 |
| Sample No.3 | Puff | 14.5 | 9.52 | 3.01 | 0.91 | 13.44 | 92.69 |
| Average | | | | | | | **91.24** |

[Table 3]

| Comp.Ex. 1 | Applicator | Loaded amount (g) | Stage 1 dispense d amount (g) | Stage 2 dispensed amount (g) | Stage 3 dispensed amount (g) | Total dispense d amount (g) | Dispensed amount / Loaded amount (%) |
|---|---|---|---|---|---|---|---|
| Sample No.1 | Puff | 14.5 | 8.95 | 1.79 | 1.31 | 12.05 | 83.10 |
| Sample No.2 | Puff | 14.5 | 8.69 | 1.95 | 1.95 | 12.59 | 86.83 |
| Sample No.3 | Puff | 14.5 | 9.26 | 2.06 | 0.65 | 11.97 | 82.55 |
| Average | | | | | | | **84.16** |

[Table 4]

| Comp.Ex. 2 | Applicator | Loaded amount (g) | Stage 1 dispensed amount (g) | Stage 2 dispensed amount (g) | Stage 3 dispensed amount (g) | Total dispensed amount (g) | Dispensed amount / Loaded amount (%) |
|---|---|---|---|---|---|---|---|
| Sample No.1 | Puff | 14.5 | 5.2 | 2.04 | 2.85 | 10.09 | 69.59 |
| Sample No.2 | Puff | 14.5 | 3.39 | 2.1 | 4.25 | 9.74 | 67.17 |
| Sample No.3 | Puff | 14.5 | 5.8 | 2.55 | 2.8 | 11.15 | 76.90 |
| Average | | | | | | | **71.22** |

[Table 5]

| Comp.Ex. 3 | Applicator | Loaded amount (g) | Stage 1 dispensed amount (g) | Stage 2 dispensed amount (g) | Stage 3 dispensed amount (g) | Total dispensed amount (g) | Dispensed amount / Loaded amount (%) |
|---|---|---|---|---|---|---|---|
| Sample No.1 | Puff | 14.5 | 9.01 | 2.72 | 0.94 | 12.67 | 87.38 |
| Sample No.2 | Puff | 14.5 | 9.19 | 2.54 | 0.7 | 12.43 | 85.72 |
| Sample No.3 | Puff | 14.5 | 9.21 | 2.51 | 0.85 | 12.57 | 86.69 |
| Average | | | | | | | **86.60** |

As can be seen from the results of Tables 2-5, Comparative Example 2 including rubber sponge having a pore size of 0.15 mm and density of 330 g/L and impregnation with emulsion containing both an inorganic ultraviolet blocking agent and an organic ultraviolet blocking agent shows the lowest usage efficiency of about 71%. On the contrary, Example 1 including rubber sponge having a pore size of 0.5 mm and density of 200 g/L and impregnated with emulsion containing an inorganic ultraviolet blocking agent only shows the highest efficiency of about 91%. It can be seen that the difference in usage efficiency is about 20%.

**Test Example 2: Determination of Analyzed Content of Sunscreen Based on Formulated Content of Sunscreen**

The foundation according to each of Example 1 and Comparative Examples 1-3 was impregnated in sponge and stored in a constant-temperature container at 25°C for at least one day.

First, the sponge impregnated with each of the four types of foundation samples was squeezed by the hands to collect foundation.

According to Korean Functional Cosmetics Codex (KFCC) 2013-28 notified by the Ministry of Food and Drug Safety of Korea, each of the foundation before being impregnated in the sponge and the foundation collected from the sponge was dissolved in a solvent and analyzed by liquid chromatography equipped with a UV absorption spectrometer to obtain peak area AT of ethylhexylmethoxy cinnamate (EHMC) and ethylhexyl salicylate (EHS) and peak area AS of the standard product, and to analyze a change between before and after impregnation.

In addition, titanium dioxide ($TiO_2$) was analyzed by introducing each of the foundation before being impregnated in the sponge and the foundation collected from the sponge to acid to carry out pretreatment, and then carrying out a test by using an inductive coupled plasma spectrometer. Then, a change between before and after impregnation was calculated and analyzed according to the following Formula 1 to Formula 3. The results are shown in the following Table 6.

[Formula 1]

Amount of ethylhexylmethoxy cinnamate (mg) = AT/AS x amount of ethylhexylmethoxy cinnamate standard (mg) x 1/10

[Formula 2]

Amount of ethylhexy salicylate (mg) = AT/AS x amount of ethylhexyl salicylate standard (mg) x 1/10

[Formula 3]

Amount of titanium dioxide (%) = (Concentration measurement of Ti (ppm) x sample liquid volume (mL) x 1.668494) / (collected sample amount (g) x 10000)

[Table 6]

|  | Analyzed Content (% based on Formulated Content) | | |
|---|---|---|---|
|  | EHMC | EHS | Titanium dioxide |
| Example1 | - | - | 11.63 (101.3%) |
| Comp.Ex.1 | 4.36 (58.1%) | 2.77 (69.3%) | 11.71 (102%) |
| Comp.Ex.2 | 4.25 (56.6%) | 2.65 (66.2%) | 11.83 (103%) |
| Comp.Ex.3 | 4.79 (63.8%) | 2.95 (73.7%) | 11.75 (102%) |

After analyzing the amount of organic ultraviolet blocking agent dispensed from the sponge based on the amount of formulated organic ultraviolet blocking agent, the amount of ethylhexymethoxy cinnamate is 59.5% on average and that of ethylhexyl salicylate is 69.7% on average. Thus, it can be seen that the organic ultraviolet blocking agents are adsorbed to the rubber sponge, and thus only a significantly decreased amount of organic ultraviolet blocking agents is dispensed. On the contrary, in the case of titanium dioxide as an inorganic ultraviolet blocking agent, 100% of the formulated amount is analyzed. As a result, it can be seen that the inorganic ultraviolet blocking agent is not adsorbed to the rubber sponge but is dispensed wholly during use as compared to the formulated amount.

**Test Example 3: Formulation Stability Test**

Each of the four types of foundation as shown in Table 1 was tested for formulation stability under the conditions as shown in the following Table 7. The results are shown in Table 7.

[Table 7]

|  | 45°C 15-day observation | Cycle (-10°C → 15°C → 40°C each 8 hours) 5-day observation |
|---|---|---|
| Example1 | stable | stable |
| Comp.Ex.1 | stable | stable |
| Comp.Ex.2 | stable | stable |
| Comp.Ex.3 | separation of content | separation of content |

It can be seen from Table 7 that when the W/O emulsion make-up cosmetic composition according to the present invention is impregnated in rubber sponge having a different pore size and density, and the cosmetic products are compared to one another in terms of stability, Comparative Example 3 using rubber sponge having a pore size of 1.2 mm on average is instable, since it shows separation of the formulation in each constant-temperature container.

**Claims**

1. A sunscreen cosmetic product including a foamed rubber sponge and a cosmetic composition impregnated in the foamed rubber sponge, wherein the cosmetic composition comprises only an inorganic ultraviolet blocking agent as an ultraviolet blocking agent, and comprises no organic ultraviolet blocking agent.

2. The sunscreen cosmetic product according to claim 1, wherein the foamed rubber sponge has a pore size of 0.1-1.0 mm, density of 150-250 g/L and a hardness of 22 $\pm$ 5 based on Asker hardness F.

3. The sunscreen cosmetic product according to claim 1, wherein the foamed rubber sponge is styrene-butadiene rubber (SBR), butadiene rubber (BR), acrylonitrile rubber (NR) or acrylonitrile-butadiene rubber (NBR).

4. The sunscreen cosmetic product according to claim 1, wherein the cosmetic composition impregnated in the foamed rubber sponge has a viscosity of 4,000-15,000 cps.

5. The sunscreen cosmetic product according to claim 1, wherein the cosmetic composition is make-up base, blemish balm (BB) cream, foundation, primer or sunscreen.

## INTERNATIONAL SEARCH REPORT

| International application No. |
| --- |
| **PCT/KR2017/004876** |

**A. CLASSIFICATION OF SUBJECT MATTER**

*A61K 8/04(2006.01)i, A61K 8/29(2006.01)i, A61K 8/27(2006.01)i, A61Q 17/04(2006.01)i*

According to International Patent Classification (IPC) or to both national classification and IPC

**B. FIELDS SEARCHED**

Minimum documentation searched (classification system followed by classification symbols)
A61K 8/04; A61K 8/02; A61K 8/89; A61K 8/65; A61K 8/27; A61K 8/40; A61K 8/29; C08G 18/00; C08J 9/00; A61K 8/19; A61Q 17/04

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)
eKOMPASS (KIPO internal) & Keywords: foam-rubber sponge, inorganic UV blocker, asker durometer, SBR, BR, NR, NBR

**C. DOCUMENTS CONSIDERED TO BE RELEVANT**

| Category* | Citation of document, with indication, where appropriate, of the relevant passages | Relevant to claim No. |
| --- | --- | --- |
| X | KR 10-2017-0012056 A (AMOREPACIFIC CORPORATION) 02 February 2017 See paragraphs [0006], [0040], [0044], [0048]-[0050]; claims 1, 14, 16. | 1-5 |
| A | KR 10-2015-0117619 A (AMOREPACIFIC CORPORATION) 20 October 2015 See abstract; claims 1-13. | 1-5 |
| A | KR 10-1542917 B1 (LG HOUSEHOLD & HEALTH CARE LTD.) 07 August 2015 See the entire document. | 1-5 |
| A | KR 10-2009-0070454 A (COREANA COSMETICS CO., LTD.) 01 July 2009 See the entire document. | 1-5 |
| A | KR 10-2012-0065982 A (AMOREPACIFIC CORPORATION) 21 June 2012 See the entire document. | 1-5 |
| A | KR 10-1551370 B1 (SEOUL COSMETIES, LTD.) 09 September 2015 See the entire document. | 1-5 |
| E | KR 10-2017-0052715 A (COSMAX, INC.) 15 May 2017 See abstract; claims 1-6. | 1-5 |

| ☐ Further documents are listed in the continuation of Box C. | ☒ See patent family annex. |
| --- | --- |

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
| --- | --- |
| 18 JANUARY 2018 (18.01.2018) | **18 JANUARY 2018 (18.01.2018)** |

| Name and mailing address of the ISA/KR | Authorized officer |
| --- | --- |
| Korean Intellectual Property Office Government Complex Daejeon Building 4, 189, Cheongsa-ro, Seo-gu, Daejeon, 35208, Republic of Korea Facsimile No. +82-42-481-8578 | Telephone No. |

Form PCT/ISA/210 (second sheet) (January 2015)

INTERNATIONAL SEARCH REPORT
Information on patent family members

International application No.

**PCT/KR2017/004876**

| Patent document cited in search report | Publication date | Patent family member | Publication date |
|---|---|---|---|
| KR 10-2017-0012056 A | 02/02/2017 | WO 2017-014519 A1 | 26/01/2017 |
| KR 10-2015-0117619 A | 20/10/2015 | CA 2944694 A1 | 15/10/2015 |
| | | CN 106456456 A | 22/02/2017 |
| | | EP 3130326 A1 | 15/02/2017 |
| | | JP 2017-510616 A | 13/04/2017 |
| | | KR 10-1562221 B1 | 23/10/2015 |
| | | KR 10-2015-0117623 A | 20/10/2015 |
| | | KR 10-2015-0119832 A | 26/10/2015 |
| | | US 2017-0112752 A1 | 27/04/2017 |
| | | WO 2015-156650 A1 | 15/10/2015 |
| KR 10-1542917 B1 | 07/08/2015 | EP 3072500 A1 | 28/09/2016 |
| | | JP 2016-130237 A | 21/07/2016 |
| | | JP 2017-088631 A | 25/05/2017 |
| | | JP 6113871 B2 | 12/04/2017 |
| | | KR 10-1691532 B1 | 30/12/2016 |
| | | KR 10-1694460 B1 | 09/01/2017 |
| | | KR 10-2016-0086728 A | 20/07/2016 |
| | | KR 10-2016-0092899 A | 05/08/2016 |
| | | KR 10-2017-0001700 A | 04/01/2017 |
| | | US 2016-0331648 A1 | 17/11/2016 |
| | | WO 2016-114466 A1 | 21/07/2016 |
| KR 10-2009-0070454 A | 01/07/2009 | KR 10-0968715 B1 | 08/07/2010 |
| KR 10-2012-0065982 A | 21/06/2012 | CA 2718712 A1 | 24/09/2009 |
| | | CA 2718712 C | 27/10/2015 |
| | | CA 2900091 A1 | 24/09/2009 |
| | | CN 101977587 A | 16/02/2011 |
| | | CN 101977587 B | 14/11/2012 |
| | | KR 10-1159877 B1 | 25/06/2012 |
| | | KR 10-1355051 B1 | 27/01/2014 |
| | | KR 10-2009-0100643 A | 24/09/2009 |
| | | US 2011-0014254 A1 | 20/01/2011 |
| | | US 2013-0045258 A1 | 21/02/2013 |
| | | US 2013-0045259 A1 | 21/02/2013 |
| | | US 8784854 B2 | 22/07/2014 |
| | | WO 2009-116817 A2 | 24/09/2009 |
| | | WO 2009-116817 A3 | 10/12/2009 |
| KR 10-1551370 B1 | 09/09/2015 | KR 10-2015-0011887 A | 03/02/2015 |
| KR 10-2017-0052715 A | 15/05/2017 | KR 10-1743436 B1 | 16/06/2017 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• KR 1542917